# EUROPEAN PATENT APPLICATION

(11) **EP 2 138 089 A1**
(43) Date of publication of application: **30.12.2009**
(21) Application number: 09007955.9
(22) Date of filing: 17.06.2009
(51) Int. Cl.: A61B 1/005

(54) **Flexible tube device and endoscope**

(30) Priority: 23.06.2008 JP 2008163453
(71) Applicant: FUJIFILM Corporation, Minato-ku Tokyo (JP)
(72) Inventor: Yoshimoto, Yoshiyuki, Kita-ku Saitama-shi Saitama (JP)
(74) Representative: Klunker . Schmitt-Nilson . Hirsch

(57) **Abstract**

A flexible tube device has proximal and distal ends (21a, 21b), for attachment of the proximal end to a handle assembly (12) of an endoscope (10). The flexible tube device includes a spiral pipe (18) formed by helically winding an elongate strip of metal. A tubular mesh material (19) is disposed around the spiral pipe. A jacket material (22, 51) is disposed around the tubular mesh material, and formed from rigid resin (24, 52) and non-rigid resin (25, 53). In the jacket material, a content of the non-rigid resin is higher than a content of the rigid resin at the proximal end. The content of the rigid resin gradually increases from the proximal end toward a first change point (26, 54) predetermined nearer to the proximal end than to the distal end. The content of the rigid resin is higher than the content of the non-rigid resin at the first change point.

## Description

### BACKGROUND OF THE INVENTION

### 1. Field of the Invention

The present invention relates to a flexible tube device and endoscope. More particularly, the present invention relates to a flexible tube device and endoscope in which a jacket material of a tubular structure can be free from buckling or other incidental errors even when an operator turns the tubular structure by twisting operation.

### 2. Description Related to the Prior Art

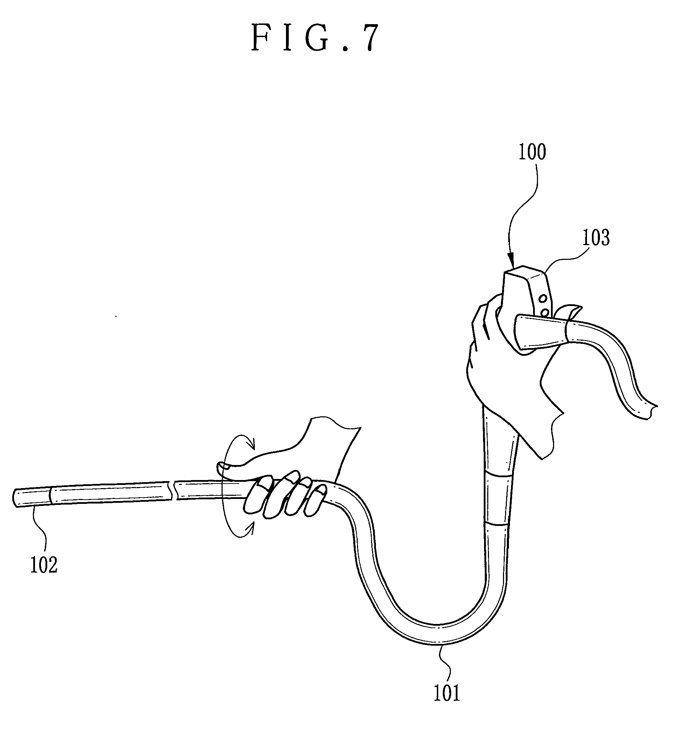

An endoscope for medical use is known as a device for in-vivo imaging of an organ in a patient's body. In Fig. 7, an endoscope 100 includes an insertion tube 101 and a head assembly 102. The insertion tube 101 is entered in the body. The head assembly 102 of the insertion tube 101 includes an image pickup unit, imaging optical system and the like. A handle assembly 103 is connected with a proximal end of the insertion tube 101 for an operator to operate the endoscope.

A flexible tube device is prepared to constitute the insertion tube 101 for advancing the head assembly 102 toward an object of interest through a gastrointestinal tract with tortuous paths. A main portion of the flexible tube device except for the head assembly 102 is all flexible. The flexible tube device includes a tubular structure or tube material, and jacket material or sheath layer or cladding. The tubular structure includes a spiral pipe or tubular coil, and tubular mesh material. The spiral pipe is created by winding a strip of metal. The tubular mesh material is disposed about the spiral pipe. The jacket material is formed from thermoplastic resin such as polyurethane resin, and disposed about the tubular structure. If the steerability into the body is considered, flexibility of the flexible tube device should be high. However, a flexible structure of a full length of the flexible tube device has a problem in the difficulty of operating force of an operator from a proximal end of the flexible tube device toward a distal end. In view of this, JP-U 55-112505 discloses the flexible tube device in which a portion of the distal end has high flexibility, and of which hardness increases toward the proximal end. The hardness of the flexible tube device changes in the longitudinal direction and maximized at the proximal end.

In Fig. 7, an example of handling of the endoscope 100 by an operator is illustrated. When the insertion tube 101 is entered in the gastrointestinal tract, a portion of the insertion tube 101 outside the body is grasped by his or her right hand. The handle assembly 103 is held by his or her left hand and kept stationary. Only the insertion tube 101 is twisted by the right hand about the axis of the core. This is the twisting operation which a certain operator carries out to change the direction of the head assembly 102. A portion of the insertion tube 101 near to the proximal end is twisted relative to the handle assembly 103 by twisting operation. In the flexible tube device disclosed in JP-U 55-112505, there is a problem of difficult twisting due to hardness of the portion near to the proximal end.

JP-U 58-108801 discloses the flexible tube device in which a plurality of jacket materials with different hardness levels are connected with one another for higher flexibility in the vicinity of the proximal and distal ends in comparison with a middle portion of the insertion tube 101. This facilitates the twisting operation of the proximal end owing to the high flexibility of the proximal end, to raise the operability.

However, the flexible tube device of JP-U 58-108801 is constructed by connection of a plurality of jacket materials which are different in the hardness. There are abrupt changes between the jacket materials in the hardness with considerable discontinuity on their borderlines. If high stress is applied to the borderlines by the twisting operation, the jacket materials are likely to buckle or tear seriously.

### SUMMARY OF THE INVENTION

In view of the foregoing problems, an object of the present invention is to provide a flexible tube device and endoscope in which a jacket material of a tubular structure can be free from buckling or other incidental errors even when an operator turns the tubular structure by twisting operation.

In order to achieve the above and other objects and advantages of this invention, a flexible tube device is provided, having proximal and distal ends, for attachment of the proximal end to a handle assembly of an endoscope. A spiral pipe is formed by helically winding an elongate strip of metal. A tubular mesh material is disposed around the spiral pipe. A jacket material is disposed around the tubular mesh material, and contains rigid resin and non-rigid resin. In the jacket material, a content of the non-rigid resin is higher than a content of the rigid resin at the proximal end, and the content of the rigid resin gradually increases from the proximal end toward a first change point predetermined nearer to the proximal end than to the distal end, and the content of the rigid resin is higher than the content of the non-rigid resin at the first change point.

In the jacket material, the content of the non-rigid resin gradually increases from the first change point toward the distal end, and is higher than the content of the rigid resin at the distal end.

In one preferred embodiment, the jacket material, the contents of the rigid and non-rigid resins at the first change point are maintained between the first change point and a second change point which is predetermined nearer to the distal end than to the proximal end. The content of the non-rigid resin gradually increases from the second change point toward the distal end, and is higher than the content of the rigid resin at the distal end.

A distance between the second change point and the distal end is longer than a distance between the first change point and the proximal end.

The jacket material includes a high hardness layer formed from the rigid resin, and a low hardness layer formed from the non-rigid resin.

The contents of the rigid and non-rigid resins change according to a change in thicknesses of the high and low hardness layers.

The high hardness layer is overlaid on the tubular mesh material, and the low hardness layer is overlaid on the high hardness layer.

The rigid and non-rigid resins are thermoplastic elastomer.

The jacket material is formed by extrusion.

In one aspect of the invention, an endoscope is provided, and includes a handle assembly. A flexible tube device has proximal and distal ends, the proximal end being attached to the handle assembly. The flexible tube device includes a spiral pipe formed by helically winding an elongate strip of metal. A tubular mesh material is disposed around the spiral pipe. A jacket material is disposed around the tubular mesh material, and formed from rigid resin and non-rigid resin. In the jacket material, a content of the non-rigid resin is higher than a content of the rigid resin at the proximal end, and the content of the rigid resin gradually increases from the proximal end toward a first change point predetermined nearer to the proximal end than to the distal end, and the content of the rigid resin is higher than the content of the non-rigid resin at the first change point.

Furthermore, there is a head assembly for endoscopic imaging. A steering assembly is connected between the head assembly and the distal end of the flexible tube device, actuated by a steering operation, for changing a direction of extension.

Consequently, a jacket material of a tubular structure can be free from buckling or other incidental errors even when an operator turns the tubular structure by twisting operation, because of the balanced use of the rigid and non-rigid resins in the jacket material.

### BRIEF DESCRIPTION OF THE DRAWINGS

The above objects and advantages of the present invention will become more apparent from the following detailed description when read in connection with the accompanying drawings, in which:
Fig. 1 is a top plan illustrating an electronic endoscope;
Fig. 2 is a vertical section illustrating a first preferred flexible tube device;
Fig. 3 is a block diagram, partially in a section, illustrating a continuous extruding apparatus;
Fig. 4 is a graph illustrating a change in a moving speed in a first extruding step;
Fig. 5 is a graph illustrating a change in a moving speed in a second extruding step;
Fig. 6 is a vertical section illustrating a second preferred flexible tube device; and
Fig. 7 is a perspective view illustrating a state of an endoscope in a twisting operation of an operator.

### DETAILED DESCRIPTION OF THE PREFERRED

### EMBODIMENT(S) OF THE PRESENT INVENTION

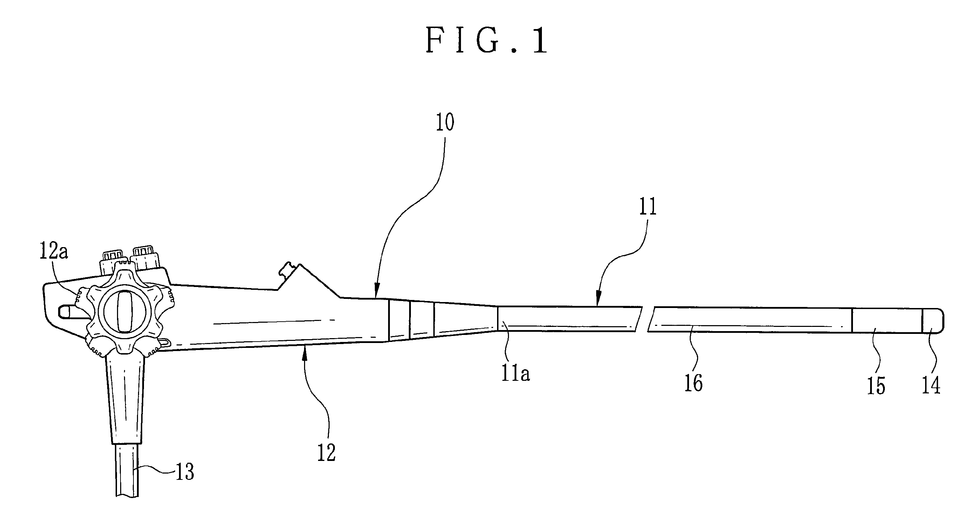

In Fig. 1, an electronic endoscope 10 including a flexible tube device of the invention is illustrated. The endoscope 10 includes an insertion tube 11 and a handle assembly 12. The insertion tube 11 is entered orally in a gastrointestinal tract of a patient's body. The handle assembly 12 is connected with a proximal end 11a of the insertion tube 11. A universal cable 13 of a flexible form extends from the handle assembly 12, and is removably connected to a processor (not shown) as external device.

The insertion tube 11 includes a head assembly 14, a steering assembly 15, and a bendable portion 16 arranged in a direction from the distal side. The head assembly 14 includes an image pickup device (not shown) for in-vivo imaging. The steering assembly 15 extends form the head assembly 14 and is steerable for changing a direction of the head assembly 14. The bendable portion 16 extends from the steering assembly 15 to the handle assembly 12, and is a main portion of the insertion tube 11 as long as approximately 1.3-1.7 meters.

The head assembly 14 includes an objective lens, CCD, lighting lens, and a lighting LED (light emitting diode). The objective lens receives image light from an object in the patient's body. The CCD detects the image light to form an image. The LED applies light through the lighting lens to the object. An endoscopic image is obtained by the CCD, and output and displayed on a display panel connected with the processor.

A steering wheel 12a is rotatable on the handle assembly 12. A wire through the insertion tube 11 is moved back and forth when the steering wheel 12a is rotated, to shift the steering assembly 15 up and down and to the right and left by curving. Thus, the head assembly 14 is directed as desired within the patient's body.

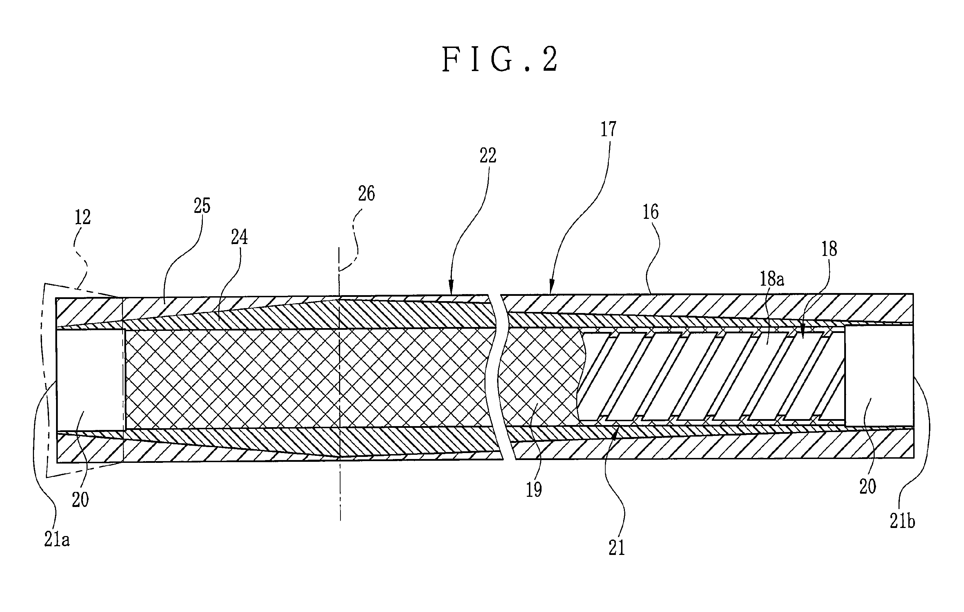

In Fig. 2, a flexible tube device 17 constitutes the bendable portion 16. The flexible tube device 17 includes a tubular structure 21 or tube material, and a jacket material 22 or sheath layer or cladding. The tubular structure 21 includes a spiral pipe or tubular coil 18, a tubular mesh material 19, and seal rings 20. The spiral pipe or tubular coil 18 is formed by helically winding an elongate strip 18a of metal. The tubular mesh material 19 is created by use of wires of metal, and is fitted around the spiral pipe 18. The seal rings 20 are attached to distal ends of the tubular mesh material 19, to obtain the tubular structure 21. The jacket material 22 is overlaid on a peripheral surface of the tubular structure 21.

The jacket material 22 has a dual layer structure, and is constituted by rigid resin 24 overlaid on the outside of the tubular structure 21, and non-rigid resin 25 overlaid on the outside of the layer of the rigid resin 24. The rigid and non-rigid resins 24 and 25 are thermoplastic elastomers with different hardness levels. Note that in the drawings, the layer thicknesses of the jacket material 22 and the rigid and non-rigid resins 24 and 25 are depicted in an exaggerated manner for the purpose of clarification.

The tubular structure 21 has a proximal end 21a and a distal end 21b. The proximal end 21a is positioned on the handle assembly 12. There is a change point 26 defined at a distance of approximately 10-20 cm from the proximal end 21a. A layer thickness of the rigid resin 24 is set the greatest at the change point 26, gradually decreases toward the proximal end 21a and toward the distal end 21b, and is set the smallest at the proximal and distal ends 21a and 21b. Thus, the rigid resin 24 has the layer thickness with a distribution of one peak. A section of the tubular structure 21 from the proximal end 21a to the change point 26 is normally a portion without entry in a patient's body in diagnosis of a gastrointestinal tract. A total length of the flexible tube device 17 is approximately 130-170 cm. The change point 26 is nearer to the proximal end 21a than to the distal end 21b. Note that the steering assembly 15 is secured to the distal end 21b of the flexible tube device 17.

The layer thickness of the non-rigid resin 25 is the smallest at the change point 26, gradually increases from the change point 26 toward the proximal end 21a and toward the distal end 21b, and is the greatest at the proximal and distal ends 21a and 21b. The distribution of the layer thickness of the non-rigid resin 25 is opposite to that of the rigid resin 24 in relation to the inclination. The rigid and non-rigid resins 24 and 25 are present with a constant sum of their layer thicknesses for the purpose of regularizing the diameter of the jacket material 22 in the full length of the flexible tube device 17.

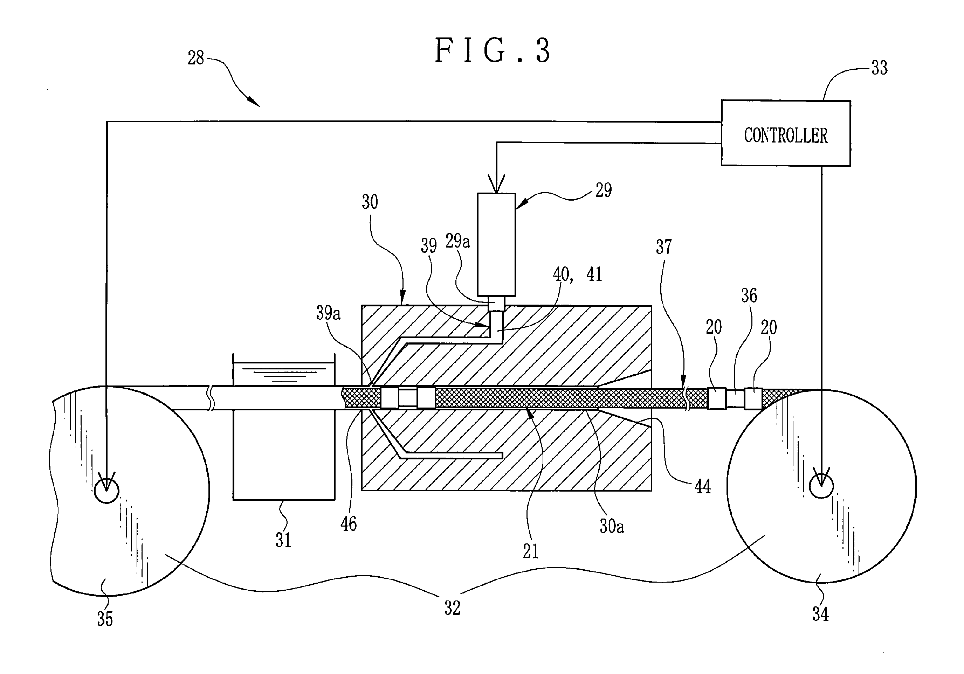

In Fig. 3, a continuous extruding apparatus 28 is illustrated, and includes an extruder screw barrel 29, an extrusion head 30, a cooler 31, a moving device 32 and a controller 33. The extruder screw barrel 29 is a device well-known in the art, and associated with a hopper and the like. The extrusion head 30 has an axial passage 30a which is circular as viewed in a section, and where the tubular structure 21 is moved. The extrusion head 30 forms the jacket material 22 by extruding molten resin from the extruder screw barrel 29 to the peripheral surface of the tubular structure 21. The cooler 31 cools the jacket material 22. The moving device 32 moves the tubular structure 21. The controller 33 controls those various elements in the continuous extruding apparatus 28.

The moving device 32 includes a supply drum 34 and a winding drum 35. Continuous tubing 37 or tube material is wound about the supply drum 34. In the continuous tubing 37, a joint structure 36 is used to interconnect the tubular structures 21. The layer thicknesses of the rigid and non-rigid resins 24 and 25 are equal between the positions of the proximal and distal ends 21a and 21b. No adjustment of the moving speed of the continuous tubing 37 is required in passage of the joint structure 36 in connection of the distal end 21b to the proximal end 21a of the tubular structure 21. The joint structure 36 in the invention can have a smaller length than that used conventionally. Thus, the cost of the flexible tube device 17 can be reduced by reducing the amount of waste resin of a coating applied to the joint structure 36.

The continuous tubing 37 unwound from the supply drum 34 is passed through the axial passage 30a in the extrusion head 30 and the cooler 31, and becomes wound by the winding drum 35. Rotations of the supply drum 34 and the winding drum 35 are controlled by the controller 33, for changing the moving speed of moving the continuous tubing 37. As will be described later, layer thicknesses of the rigid and non-rigid resins 24 and 25 are controlled by changes in the moving speed for distribution with an increasing and decreasing level.

A passage or nozzle 29a at an adapter extends from the extruder screw barrel 29. An annular channel 39 or gate with a manifold is formed in the extrusion head 30. The passage 29a is connected with the annular channel 39. Also, an annular orifice 39a is formed in the extrusion head 30 at the end of the annular channel 39. Molten rigid resin 40 is supplied by the extruder screw barrel 29 and extruded through the annular orifice 39a into the axial passage 30a at a constant pressure of extrusion. After the extrusion, the molten rigid resin 40 is removed from the extruder screw barrel 29. Molten non-rigid resin 41 is filled in the extruder screw barrel 29, and is extruded through the annular orifice 39a at a constant pressure.

Note that the resins for extrusion can be changed over not by alternate use of the resins in the single extruder screw barrel 29 but by selective use of two suitable extruder screw barrels 29. Specifically, a first one of the extruder screw barrels 29 is removed before positioning a second one of the extruder screw barrels 29. Alternatively, two extruder screw barrels 29 can be incorporated in the extruder, and contain respectively the molten rigid and non-rigid resins 40 and 41. A selecting mechanism may drive the two extruder screw barrels 29 to extrude the molten rigid resin 40 at first and then extrude the molten non-rigid resin 41.

A guide channel 44 is formed in the extrusion head 30, and has a conical shape, for guiding the continuous tubing 37 to facilitate insertion in the axial passage 30a. Bores of the axial passage 30a and an exit opening 46 are determined according to an outer diameter of the jacket material 22 formed around the tubular structure 21.

After applying the coating of the molten rigid resin 40 to the continuous tubing 37, the continuous tubing 37 is passed out of the extrusion head 30 and moved to the cooler 31. Cooling water or other coolant is stored in the cooler 31. The molten rigid resin 40 is cooled and hardened by passage of the continuous tubing 37 in the cooling water, to form the rigid resin 24. Similarly, the molten non-rigid resin 41 is cooled by the cooler 31 and hardened to form the non-rigid resin 25. Note that any suitable one of various cooling methods can be used for cooling the molten rigid and non-rigid resins 40 and 41, for example, blowing cooling liquid or cooling air to the molten resin.

A sequence of forming the jacket material 22 on the continuous tubing 37 in the continuous extruding apparatus 28 is described now. In a first extruding step for the rigid resin 24, the continuous tubing 37 is unwound from the supply drum 34 and drawn through the axial passage 30a. A distal end of the continuous tubing 37 is engaged with the winding drum 35.

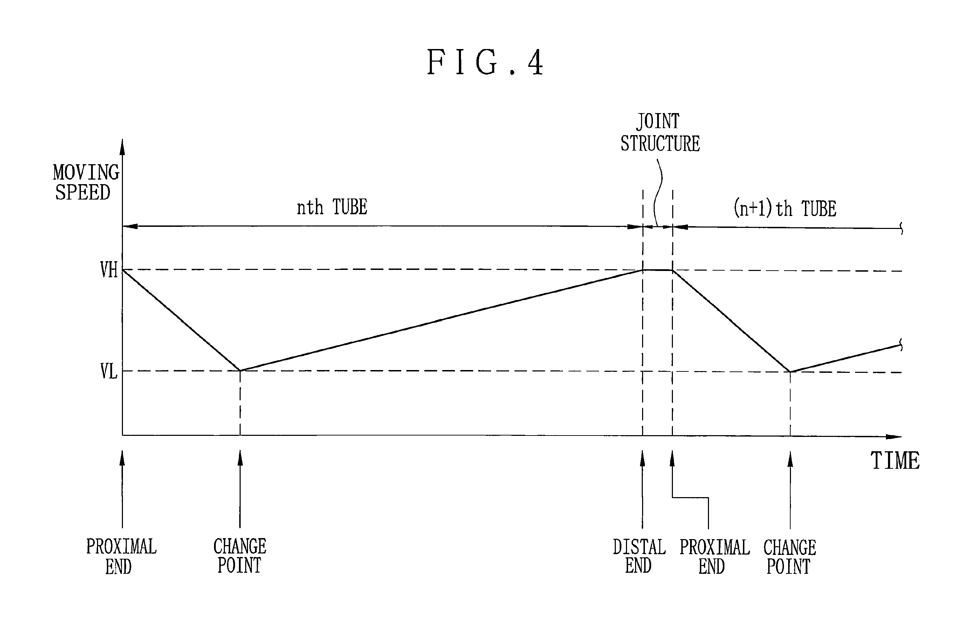

The supply drum 34 and the winding drum 35 are controlled by the controller 33 for rotations, to control the moving speed of the continuous tubing 37. In Fig. 4, the moving speed becomes the highest speed VH at the proximal end 21a of the nth tubular structure 21, and decreases from the proximal end 21a to the change point 26 with a great acceleration rate which is constant. The moving speed becomes the lowest speed VL at the change point 26. The moving speed increases from the change point 26 to the distal end 21b with a smaller acceleration rate than the acceleration rate from the proximal end 21a to the change point 26. Thus, the moving speed gradually changes from the lowest speed VL to the highest speed VH at the distal end 21b. In the joint section from the distal end 21b to the proximal end 21a of next tubular structure 21, the moving speed is the highest speed VH constantly. After this, the speed control for an (n+1)th tubular structure 21 is carried out in the similar manner to the nth tubular structure 21.

While the continuous tubing 37 is moved, the molten rigid resin 40 is supplied through the passage 29a of the extruder screw barrel 29, and extruded through the annular orifice 39a of the annular channel 39 for ejection into the axial passage 30a of the extrusion head 30. The pressure of the extrusion is kept constant by the control of the controller 33.

The moving speed of the continuous tubing 37 is changed as indicated in Fig. 4 under the pressure of extrusion of the molten rigid resin 40. A layer thickness of the rigid resin 24 is the smallest at the proximal end 21a of the tubular structure 21 as illustrated in Fig. 2, but increases from the proximal end 21a toward the change point 26. The rigid resin 24 has the greatest layer thickness at the change point 26. The layer thickness of the rigid resin 24 decreases gradually from the change point 26 toward the distal end 21b, and is the smallest at the distal end 21b. In short, distribution of the layer thickness of the rigid resin 24 about the tubular structure 21 is in the form of an inverted V with a peak at the change point 26 as viewed in a graph. Note that the rigid resin 24 has the smallest layer thickness in the joint section in the manner similar to the distal end 21b.

Note that a moving speed of the continuous tubing 37 is experimentally obtained by test extrusion of the rigid resin 24 in the first extruding step so as to determine layer thicknesses and forms of various layer portions of the rigid resin 24. To this end, the pressure of extruding the molten rigid resin 40 is constant in the extruder screw barrel 29. Also, a moving speed of the continuous tubing 37 in the second extruding step is experimentally obtained, which will be described later.

Then the second extruding step for the non-rigid resin 25 is started. The continuous tubing 37 with the rigid resin 24 applied in the first extruding step is removed from the winding drum 35 in a roll form. The roll of the continuous tubing 37 is set on the supply drum 34 to advance the distal end 21b of the tubular structure 21 from the supply drum 34 as a leading end of the continuous tubing 37. Then the continuous tubing 37 is unwound from the supply drum 34. The leading end of the continuous tubing 37 is engaged with the winding drum 35 after passage of the continuous tubing 37 through the axial passage 30a.

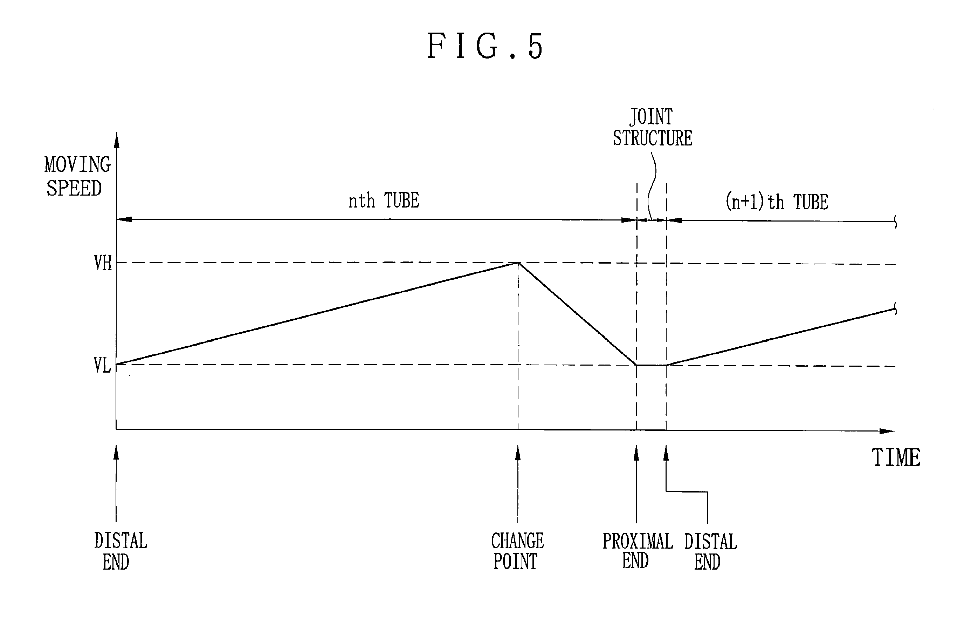

The moving speed of the continuous tubing 37 is controlled by the controller 33. In Fig. 5, the moving speed becomes the lowest speed VL at the distal end 21b of the nth of the tubular structures 21, and gradually increases from the distal end 21b to the change point 26 at a regular acceleration rate, and becomes the highest speed VH at the change point 26. The moving speed decreases from the highest speed VH to the to the lowest speed VL between the change point 26 and the proximal end 21a at a regular acceleration rate which is much higher than that between the distal end 21b and the change point 26. At the proximal end 21a, the moving speed becomes the lowest speed VL. In the section of the joint structure from the proximal end 21a to the distal end 21b of next tubular structure 21, the moving speed is the lowest speed VL constantly. Thus, the speed is controlled in the (n+1) th tubular structure 21 in a manner similar to the nth tubular structure 21.

Note that the proximal end 21a of the tubular structure 21 can be advanced from the supply drum 34 as a leading end of the continuous tubing 37 in the second extruding step in a manner similar to the first extruding step. A graph of the moving speed in the second extruding step is not in the form of Fig. 5 but in a form obtained by directing upside down the form of the graph of Fig. 4.

While the continuous tubing 37 is moved, the molten non-rigid resin 41 is supplied through the passage 29a of the extruder screw barrel 29, and extruded through the annular orifice 39a of the annular channel 39 into the axial passage 30a of the extrusion head 30. The pressure of the extrusion is kept constant by the controller 33.

Thus, a layer thickness of the non-rigid resin 25 around the rigid resin 24 is the greatest at the distal end 21b of the tubular structure 21, gradually decreases from the distal end 21b toward the change point 26, and becomes the smallest at the change point 26. Also, the layer thickness of the non-rigid resin 25 gradually increases from the change point 26 toward the proximal end 21a, and becomes the greatest at the proximal end 21a. In short, the layer thickness of the non-rigid resin 25 changes with a distribution with a valley at the change point 26, which is in contrast with the rigid resin 24. Note that the non-rigid resin 25 has the greatest layer thickness in the joint section in a manner similar to the proximal end 21a.

Similarly, the non-rigid resin 25 is extruded to fill portions of the rigid resin 24 with small thicknesses. Consequently, the jacket material 22 with a regular thickness is formed in relation to the full length of the continuous tubing 37. A ratio of the layer thickness of the rigid resin 24 to that of the non-rigid resin 25 is approximately 1/9 at the proximal and distal ends 21a and 21b and approximately 9/1 at the change point 26.

When the jacket material 22 is fully formed on the continuous tubing 37, the continuous tubing 37 is removed from the continuous extruding apparatus 28. The joint structure 36 is removed from the continuous tubing 37 to complete the forming of the flexible tube device 17. The flexible tube device 17 is obtained, in which the tubular structure 21 has low hardness in the vicinity of the proximal and distal ends 21a and 21b, and has an increasing hardness gradually from the distal end 21b toward the change point 26.

The bendable portion 16 is constituted by the flexible tube device 17. The insertion tube 11 of the endoscope 10 is constituted by the bendable portion 16 of the invention. As the bendable portion 16 is soft in the vicinity of the distal end and has hardness gradually increasing from the distal end toward the change point 26, it is possible to enter the endoscope in a patient's body easily with good steerability. Also, manual force of an operator can be transmitted from the proximal end of the bendable portion 16 to the distal end for the same reason. It is possible to enter the endoscope in a gastrointestinal tract in vivo even with a tortuous path. Only the insertion tube 11 can be twisted about its axis by a user's right hand while his or her left hand holds the handle assembly 12 of the endoscope 10 in a stationary manner. The twisting operation can be carried out readily because the proximal end portion of the insertion tube 11 is considerably soft. The jacket material 22 is seamless, in which no abrupt change of hardness remains. Thus, the twisting operation can be carried out safely without occurrence of buckling or tearing of the jacket material 22.

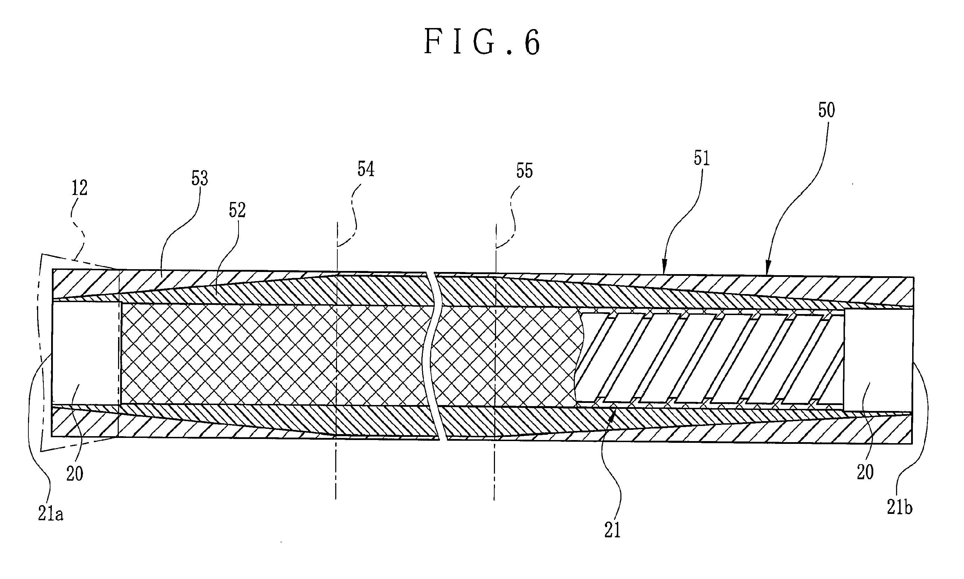

In Fig. 6, another preferred flexible tube device 50 is illustrated. A jacket material 51 or sheath layer or cladding on the flexible tube device 50 is different from the jacket material 22 of the flexible tube device 17. The jacket material 51 is constituted by rigid resin 52 and non-rigid resin 53, which are thermoplastic elastomers with different hardness levels.

A layer thickness of the rigid resin 52 is the smallest at the proximal and distal ends 21a and 21b of the tubular structure 21. Let a first change point 54 be defined at a distance of approximately 10-20 cm from the proximal end 21a. Let a second change point 55 be defined at a distance of approximately 30-40 cm from the distal end 21b. The layer thickness of the rigid resin 52 is constantly the greatest between the first and second change points 54 and 55.

In a section between the proximal end 21a and the first change point 54, the rigid resin 52 is present with a layer thickness gradually increasing from the proximal end 21a toward the first change point 54. In a section between the second change point 55 and the distal end 21b, the rigid resin 52 is present with a layer thickness gradually decreasing from the second change point 55 toward the distal end 21b.

A layer thickness of the non-rigid resin 53 is the greatest at the proximal and distal ends 21a and 21b of the tubular structure 21, and is the smallest constantly between the first and second change points 54 and 55. The layer thickness of the non-rigid resin 53 gradually decreases from the proximal end 21a toward the first change point 54, and gradually increases from the second change point 55 toward the distal end 21b. The rigid and non-rigid resins 52 and 53 are applied with a constant sum of their layer thicknesses for the purpose of regularizing the diameter of the jacket material 51 in the full length of the flexible tube device 50.

A ratio in the layer thickness between the rigid and non-rigid resins 52 and 53 is approximately 9/1 in the section between the first and second change points 54 and 55, and is approximately 1/9 at the proximal and distal ends 21a and 21b. Thus, portions of the flexible tube device 50 have high softness at the proximal and distal ends 21a and 21b. A portion of the flexible tube device 50 has low softness in the area between the first and second change points 54 and 55. An intermediate portion of the flexible tube device 50 with high hardness is approximately 53-76 % as much as the total length of the flexible tube device 50 which is approximately 130-170 cm. Thus, it is possible to transmit rotational force of a doctor or operator from the proximal side to the distal side of the bendable portion 16 for good steerability of the endoscope in the patient's body with a more remarkable feature than the first preferred embodiment. Also, the portion of the flexible tube device 50 on the proximal side is soft. The jacket material 51 is seamless, where no abrupt change of hardness remains. Thus, twisting operation of the endoscope can be carried out safely without occurrence of buckling or tearing of the jacket material 51.

Note that the ratio between those is not limited to 9/1 and 1/9 according to the above embodiments. The ratio can be determined suitably as desired for the performance of the insertion tube, for example 8/2 and 2/8, 7/3 and 3/7, or the like.

Note that the extrusion of the rigid resin may be before extrusion of the non-rigid resin, in place of the sequence of extrusion of the non-rigid resin and the rigid resin according to the embodiments. In the above embodiments, the extrudate of the rigid resin is transferred from the winding drum to the supply drum, before the non-rigid resin is extruded according to a second direction reverse to the first direction of movement. However, it is possible to prepare plural extruder screw barrels or plural extrusion heads for respectively the resins of the plural types. The extruder screw barrels or extrusion heads can be arranged in the moving direction. The tubular structure 21 may be moved in the longitudinal direction for consecutive extrusion without transfer of the tubular structure 21.

Furthermore, it is possible to form the jacket material from mixed resin containing the rigid and non-rigid resins, and to change the ratio of the mixture between those in the longitudinal direction of the flexible tube device.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A flexible tube device, having proximal and distal ends (21a, 21b), for attachment of said proximal end to a handle assembly (12) of an endoscope (10), comprising:
a spiral pipe (18) formed by helically winding an elongate strip of metal;
a tubular mesh material (19) disposed around said spiral pipe;
a jacket material (22, 51) disposed around said tubular mesh material, and containing rigid resin (24, 52) and non-rigid resin (25, 53);
wherein in said jacket material, a content of said non-rigid resin is higher than a content of said rigid resin at said proximal end, and said content of said rigid resin gradually increases from said proximal end toward a first change point (26, 54) predetermined nearer to said proximal end than to said distal end, and said content of said rigid resin is higher than said content of said non-rigid resin at said first change point.

2. A flexible tube device as defined in claim 1, wherein in said jacket material, said content of said non-rigid resin gradually increases from said first change point toward said distal end, and is higher than said content of said rigid resin at said distal end.

3. A flexible tube device as defined in claim 1, wherein in said jacket material, said contents of said rigid and non-rigid resins at said first change point are maintained between said first change point and a second change point which is predetermined nearer to said distal end than to said proximal end;
said content of said non-rigid resin gradually increases from said second change point toward said distal end, and is higher than said content of said rigid resin at said distal end.

4. A flexible tube device as defined in claim 3, wherein a distance between said second change point and said distal end is longer than a distance between said first change point and said proximal end.

5. A flexible tube device as defined in claim 1, wherein said jacket material is in a dual layer structure, and includes a high hardness layer formed from said rigid resin, and a low hardness layer formed from said non-rigid resin.

6. A flexible tube device as defined in claim 5, wherein said contents of said rigid and non-rigid resins change to cause a change in thicknesses of said high and low hardness layers.

7. A flexible tube device as defined in claim 5, wherein said low hardness layer is overlaid on said high hardness layer.

8. A flexible tube device as defined in claim 1, wherein said rigid and non-rigid resins are thermoplastic elastomer.

9. A flexible tube device as defined in claim 1, wherein said jacket material is formed by extrusion.

10. An endoscope comprising:
a handle assembly (12);
a flexible tube device, having proximal and distal ends (21a, 21b), said proximal end being attached to said handle assembly;
said flexible tube device including:
a spiral pipe (18) formed by helically winding an elongate strip of metal;
a tubular mesh material (19) disposed around said spiral pipe;
a jacket material (22, 51) disposed around said tubular mesh material, and containing rigid resin (24, 52) and non-rigid resin (25, 53);
wherein in said jacket material, a content of said non-rigid resin is higher than a content of said rigid resin at said proximal end, and said content of said rigid resin gradually increases from said proximal end toward a first change point (26, 54) predetermined nearer to said proximal end than to said distal end, and said content of said rigid resin is higher than said content of said non-rigid resin at said first change point.

11. An endoscope as defined in claim 10, wherein in said jacket material, said content of said non-rigid resin gradually increases from said first change point toward said distal end, and is higher than said content of said rigid resin at said distal end.

12. An endoscope as defined in claim 10, wherein in said jacket material, said contents of said rigid and non-rigid resins at said first change point are maintained between said first change point and a second change point which is predetermined nearer to said distal end than to said proximal end;
said content of said non-rigid resin gradually increases from said second change point toward said distal end, and is higher than said content of said rigid resin at said distal end.

13. An endoscope as defined in claim 12, wherein a distance between said second change point and said distal end is longer than a distance between said first change point and said proximal end.

14. An endoscope as defined in claim 10, wherein said jacket material is in a dual layer structure, and includes a high hardness layer formed from said rigid resin, and a low hardness layer formed from said non-rigid resin.
